# EUROPEAN PATENT APPLICATION

(11) **EP 2 420 566 A1**
(43) Date of publication of application: **22.02.2012**
(21) Application number: 10764403.1
(22) Date of filing: 09.04.2010
(51) Int. Cl.: C12N 5/07, C12Q 1/02, A61K 35/12, A61K 35/42, A61P 11/00

(54) **METHOD OF PREPARING HUMAN LUNG TISSUE STEM CELLS AND METHOD OF INDUCING DIFFERENTIATION INTO HUMAN ALVEOLAR EPITHELIAL CELLS**

(30) Priority: 17.04.2009 JP 2009100548
(71) Applicant: Tohoku University, Aoba-ku Sendai-shi Miyagi 980-8577 (JP)
(72) Inventor: KUBO, Hiroshi, Sendai-shi Miyagi 980-8577 (JP); FUJINO, Naoya, Sendai-shi Miyagi 980-8577 (JP); SUZUKI, Takaya, Sendai-shi Miyagi 980-8577 (JP); YAMAYA, Mutsuo, Sendai-shi Miyagi 980-8577 (JP)
(74) Representative: Woods, Geoffrey Corlett
(86) International application number: PCT/JP2010/056447
(87) International publication number: WO 2010/119819

(57) **Abstract**

[Abstract] Provided are: a method of preparing cells that simultaneously express a type II alveolar epithelial cell marker and a stem cell marker, including a process of isolating and extracting constituent cells from human lung tissue, and a process of separating and culturing lung tissue stem cells from the obtained isolated cells; human lung tissue stem cells that are obtained from the above method of preparation and are able to differentiate into alveolar epithelial cells; a method of inducing differentiation into human lung epithelial cells, consisting of culturing the above human lung tissue stem cells; human alveolar epithelial cells prepared through the above method of inducing differentiation; and a method of screening that uses the above human lung tissue stem cells or human alveolar epithelial cells.

## Description

### [Field of the Invention]

The present invention relates to a method of preparing human lung tissue stem cells, and a method of inducing differentiation into human alveolar epithelial cells, etc.

### [Background of the Invention]

In recent years, induced pluripotent stem cells, i.e., iPS cells, have been developed, and it has become possible to establish stem cells having genetic information unique to the patient (Patent Documents 1 and 2). There is great anticipation that the use of these iPS cells may lead to new breakthroughs in understanding clinical conditions and new drug discoveries for intractable diseases. In fact, around the world, research is being conducted toward understanding clinical conditions by establishing iPS cells from cases of intractable diseases such as muscular dystrophy. iPS cells make a significant contribution to understanding diseases in which genes are the main cause.

However, in addition to "genetic" factors, the onset of many diseases also greatly involves the factors of "environment" and "aging". Ultimately, because the establishment of iPS cells requires cell initialization caused by the introduction of "Yamanaka factors", epigenetic information generated by the environment or by aging are lost. Therefore, to understand clinical conditions and discover drugs for diseases caused by environmental and age factors, which are common in geriatric diseases, it is very important to extract tissue-specific stem cells from each organ and analyze them while keeping the epigenetic information. In other words, these types of tissue stem cells are an important key for filling in areas that cannot be covered by iPS cells.

At the same time, for tissue repair and regeneration, appropriate proliferation and differentiation of tissue-specific stem cells and precursor cells are necessary. In the reparative process of the alveolar epithelium, type II alveolar epithelial cells (type II cells) function as precursor cells for type I alveolar epithelial cells (type I cells). In other words, it is believed that after lung damage, type II cells that have proliferated differentiate into type I cells and thus cover the damaged epithelium (Non-patent Documents 1 through 6). However, type II cells are not considered stem cells that have a self-replicating function and pluripotency.

In recent years, in mouse lungs, stem cells (bronchioalveolar stem cells, or BASCs) that have self-replicating functions and the capacity to differentiate into clara cells of the bronchioles and type I and type II cells have been identified (Non-patent Document 7). Moreover, the present inventors, etc. have reported that in mouse lung damage models, stem cell groups expressing lung tissue stem cell markers proliferate after damage and are involved in the reparative process of the alveolar epithelium (Non-patent Documents 8 and 9).

Furthermore, previously reported stem cells derived from human lung tissue have included only fibroblast-like mesenchymal stem cells (Non-patent Document 10), and there are no reports of stem cells from human lung tissue that are capable of differentiating into alveolar epithelial cells.

### Prior Art Documents

### [Patent Document]

Patent Document 1: Patent No. 4183742, Specification
Patent Document 2: Japanese application publication JP 2008-307007

### [Non-patent Document]

Non-patent Document 1: Adamson, I.Y., and Bowden, D.H. 1974. The type 2 cell as progenitor of alveolar epithelial regeneration. A cytodynamic study in mice after exposure to oxygen. Lab Invest 30:35-42.
Non-patent Document 2: Evans, M.J., Cabral, L.J., Stephens, R.J., and Freeman, G. 1975. Transformation of alveolar type 2 cells to type 1 cells following exposure to NO2. Exp Mol Pathol 22:142-150.
Non-patent Document 3: Aso, Y, Yoneda, K., and Kikkawa, Y 1976. Morphologic and biochemical study of pulmonary changes induced by bleomycin in mice. Lab Invest 35:558-568.
Non-patent Document 4: Anderson, W.R., and Thielen, K. 1992. Correlative study of adult respiratory distress syndrome by light, scanning, and transmission electron microscopy. Ultrastruct Pathol 16:615-628.
Non-patent Document 5: Ware, L.B., and Matthay, M.A. 2000. The acute respiratory distress syndrome. N Engl J Med 342:1334-1349.
Non-patent Document 6: Kawanami, O., Ferrans, V.J., and Crystal, R.G. 1982. Structure of alveolar epithelial cells in patients with fibrotic lung disorders. Lab Invest 46:39-53.
Non-patent Document 7: Kim, C.F., Jackson, E.L., Woolfenden, A.E., Lawrence, S., Babar, I., Vogel, S., Crowley, D., Bronson, R.T., and Jacks, T. 2005. Identification of bronchioalveolar stem cells in normal lung and lung cancer. Cell 121:823-835.
Non-patent Document 8: Kubo, H., Hegab, A.E., He, M., Ishizawa, K., and Yamada, M. 2008. Endogenous lung stem cells increased after lung injury. Proc Am Thorac Soc 5:362-363.
Non-patent Document 9: Hegab, A.E., Kubo, H., Yamaya, M., Asada, M., He, M., Fujino, N., Mizuno, S., and Nakamura, T. 2008. Intranasal HGF administration ameliorates the physiologic and morphologic changes in lung emphysema. Mol Ther 16:1417-1426.
Non-patent Document 10: Lama, V.N., Smith, L., Badri, L., Flint, A., Andrei, A., Murray, S., Wang, Z., Liao, H., Toews, G.B., Krebsbach, P.H., Peters-Golden, M., Pinsky, D.J., Martinez, F.J., and Thannickal, V.J. 2007. Evidence for tissue-resident mesenchymal stem cells in human adult lung from studies of transplanted allografts. J Clin Invest 117:989-996

### Summary of the Invention

### Problems to be Solved by the Invention

Stem cells that are able to differentiate into the alveolar epithelial system are cells involved in tissue repair after a lung injury, and are therefore clinically very important cells for regenerative medicine, etc. Furthermore, these cells are also useful as materials for discovering new markers for identifying human lung tissue stem cells, and it is believed that analyzing the differentiation signals, etc. of these cells may lead to the discovery of new drugs.

### Means of Solving the Problems

The present inventors have discovered that stem cells that are able to differentiate into the alveolar epithelial system are present in the adult peripheral lung tissue of humans, and have succeeded in establishing methods of separating and identifying these human lung stem cells, culturing them, and inducing differentiation into the alveolar epithelium to complete the present invention.

Specifically, the present invention is related to each of the following aspects.

(Aspect 1) A method of preparing cells that simultaneously express a type II alveolar epithelial cell marker and a stem cell marker, comprising a process of isolating and extracting constituent cells from human lung tissue and a process of separating and culturing lung tissue stem cells from the obtained isolated cells.
(Aspect 2) Human lung tissue stem cells that are obtained by the above method of preparation and are able to differentiate into human alveolar epithelial cells, or human lung tissue stem cells that are obtained by passage culturing the cells.
(Aspect 3) A method of inducing differentiation into human lung epithelial cells, comprising culturing the above human lung tissue stem cells.
(Aspect 4) Human alveolar epithelial cells that are prepared by the above method of inducing differentiation, or human alveolar epithelial cells that are obtained by passage culturing the human alveolar epithelial cells.
(Aspect 5) A method of screening using the above human lung tissue stem cells or human alveolar epithelial cells.

### [Effects of the Invention]

The present invention provides a method of preparation that includes the separation and identification as well as the culturing, etc. of human lung stem cells, which are cells (e.g., SP-C+/CD90+ cells) in the adult peripheral lung tissue of humans that differentiate into the alveolar epithelial system and simultaneously express a type II alveolar epithelial cell marker and a stem cell marker, and a method of inducing differentiation from the human lung tissue stem cells into the alveolar epithelium.

### [Brief Description of the Drawings]

Fig. 1 shows graphs and photographs showing the presence of cell groups resembling mesenchymal stem cells that have the phenotype of the alveolar epithelium of a human lung.
Fig. 2 shows graphs and photographs showing that SP-C+/CD90+ cells have a self-replicating function in vitro.
Fig. 3 shows graphs and photographs showing that SP-C+/CD90+ cells have the capacity to differentiate into alveolar epithelial cells *in vitro.*
Fig. 4 shows graphs and photographs showing that SP-C+/CD90+ cells are present in the walls of the alveoli.

### Modes for Carrying Out the Invention

The first aspect of the present invention is related to a method of preparing cells (e.g., SP-C+/CD90+ cells) that simultaneously express a type II alveolar epithelial cell marker and a stem cell marker, including: a process of isolating and extracting constituent cells from human lung tissue; and a process of separating and culturing lung tissue stem cells from the obtained isolated cells. As shown in the embodiment of the present specification, these SP-C⁺/CD90⁺ cells are human lung tissue stem cells that, in addition to a self-replicating function, also have the capacity to differentiate into type I alveolar epithelial cells and type II alveolar epithelial cells through the method of inducing differentiation according to the present invention.

One preferred method for the process of isolation and extraction is a method in which, after separating and removing human lung tissue from the pleura, a solution of a suitable neutral protease (e.g., Dispase II, Dispase I (Trademark: Roche Applied Science, Mannheim, Germany), or DISPASE (Trademark: Godo Shusei Co., Ltd.), which are enzymes derived from Bacillus polymyxa (EC 3.4.24.4)) is injected into the lung tissue, and then incubation is performed in a solution containing collagenase, the neutral protease, and a suitable deoxyribonuclease such as an endonuclease like deoxyribonuclease I or deoxyribonuclease II, etc. It is particularly preferable to use Dispase II as the neutral protease injected into the lung tissue, DISPASE as the neutral protease for dissolving the cells, and deoxyribonuclease I as the deoxyribonuclease.

Furthermore, in the process of separating and culturing the lung tissue stem cells, it is preferable to perform culturing using feeder cells prepared using any method known by persons skilled in the art, particularly feeder cells composed of C57BL/6 mouse fetal fibroblasts. Suitable feeder cells that are available commercially may also be used. Moreover, for the second passage of culturing onward, it is possible to use a feeder cell conditioned medium instead of using feeder cells. Furthermore, before the above process of separation and culturing, it is preferable to remove hematopoietic cells (e.g., CD45-positive cells) from the obtained isolated cells using a suitable method known to persons skilled in the art. Human lung tissue may be obtained from parts of tissue removed during surgery, for example, upon obtaining prior consent from the patient.

It is possible to cause the human lung tissue stem cells prepared and obtained by the method of the present invention to self-replicate (proliferate) while maintaining pluripotency by performing passage culturing using a suitable method known to persons skilled in the art such as that described in the embodiment of the present specification. Consequently, the second aspect of the present invention is related to human lung tissue stem cells (e.g., SP-C⁺/CD90⁺ cells) that are obtained by the method of preparation, are able to differentiate into alveolar epithelial cells, and have a self-replicating function, or to human lung tissue stem cells that are obtained by passage culturing the cells over a suitable period.

The third aspect of the present invention is related to a method of inducing differentiation into human lung epithelial cells, comprising culturing the above human lung tissue stem cells. By performing culturing using a reconstituted basement membrane composition, it is possible to obtain type I alveolar epithelial cells within the reconstituted basement membrane composition, and to obtain type II alveolar epithelial cells from under the reconstituted basement membrane composition.

Here, the "reconstituted basement membrane composition" is obtained by being extracted and prepared from a suitable cell tissue that is contained in the thin, membranous extracellular matrix present below the cell layer *in vivo* and contains proteins and glycoproteins such as laminin, collagen IV and heparin sulphate proteoglycan as well as various cell growth factors and activating factors, etc. Examples include a soluble basement membrane preparation extracted from a mouse sarcoma such as Matrigel (trademark: BD Biosciences). For this type of reconstituted basement membrane composition, it is possible to use a material known to persons skilled in the art.

The fourth aspect of the present invention is type I alveolar epithelial cells or type II alveolar epithelial cells that are obtained by the method of inducing differentiation. Furthermore, the human lung epithelial cells that are induced to differentiate by the method of the present invention may be passage cultured over a suitable period using any method known to persons skilled in the art. Consequently, the present invention is also related to human alveolar epithelial cells that are obtained by passage culturing.

Other conditions and procedures, etc. for the method of preparation, the method of inducing differentiation, and the passage culturing of the present invention may be appropriately selected from those that are known to those skilled in the art.

The fifth aspect of the present invention is related to various methods of screening using the human lung tissue stem cells or human alveolar epithelial cells. This may be advantageously used for a method of screening substances that promote or inhibit the induction of the differentiation of the human lung tissue stem cells, for example.

The screening of the present invention may be performed using any method known to those skilled in the art.

The method of screening of the present invention may be implemented through the following processes, for example:
(a) a process of bringing the human lung tissue stem cells or human alveolar epithelial cells into contact with a test substance;
(b) a process of observing or measuring the induction of differentiation in the cells; and
(c) a process of selecting a substance that promotes or inhibits the induction of differentiation.

The present invention will now be described in detail based on an embodiment, but the technical scope of the present invention should not be interpreted as being limited in any way by the following descriptions of the embodiment. Moreover, unless specifically stated otherwise, the following embodiment has been implemented in accordance with conventional procedures in the relevant technical field and with standard methods known to those skilled in the art. Moreover, descriptions from documents cited as references in the present specification are incorporated herein as a part of the disclosure of the present specification.

### [Embodiment]

### [Methods]

Separation of human lung cells: In accordance with the past report (10), human lung cells were separated by adding several improvements. This operation was performed on a clean bench, and the instruments used were sterilized in an autoclave in advance. The provided lung tissue was cleaned using a phosphate buffer solution ((PBS, Wako Pure Chemical Industries, Osaka, Japan) containing penicillin (final concentration: 100 units/ml), streptomycin (final concentration: 100 µg/ml), and amphotericin B (final concentration: 0.25 ug/ml, Invitrogen, Carlsbad, CA). After bluntly separating the pleura, the lung tissue was cut off into a size of approximately 1 cm x 1 cm x 1 cm, 2 ml of Dispase II (final concentration: 2.0 U/ml; Roche Applied Science, Mannheim, Germany) was injected using a syringe and a 27-gauge needle, and the tissue was transferred to a 50-ml conical tube to which 8 ml of Dispase II, 1 ml of Collagenase/Dispase (final concentration: 1 mg/ml; Roche Applied Science), and 1 ml of Deoxyribonuclease I (final concentration: 0.1 mg/ml; Sigma-Aldrich, St. Luis, MO) had been added in advance and shaken for 60 minutes at 37°C. After sharply cutting the enzyme-treated lung tissue using cutting shears, 18- and 20-gauge needles were passed through 3 times each, 10 ml of a base medium added with amphotericin B (final concentration: 2.5 µg/ml) was added, and a cell suspension was prepared through a 100-µm cell strainer (BD Biosciences, San Jose, CA). The base medium was composed of Dulbecco's Modified Eagle Medium, DMEM (Invitrogen), 10% fetal bovine serum, FBS (Invitrogen), 1% amino acid (Invitrogen), penicillin (final concentration: 100 units/ml), and streptomycin (final concentration: 100 µg/ml; Sigma-Aldrich). After performing centrifugal separation on this cell suspension at 4°C and 1,500 rpm for 5 minutes, the supernatant was disposed, 3 ml of an erythrocyte hemolysis buffer solution (Roche Applied Science) was added, and a reaction was performed for 3 minutes at room temperature. After adding 10 ml of the base medium added with amphotericin B, performing centrifugal separation at 4°C and 1,500 rpm for 5 minutes, and disposing the supernatant, a similar hemolytic reaction was performed again. Then, 10 ml of the base medium added with amphotericin B was added, centrifugal separation was performed at 4°C and 1,500 rpm for 5 minutes, the supernatant was disposed, 10 ml of the base medium added with amphotericin was added, and a single cell suspension was prepared through a 40-um cell strainer (BD Biosciences).

Removal of hematopoietic cells from all lung cells: Using microbeads coated with anti-CD45 antibodies, CD45-positive cells were removed from all lung cells in a Magnetic Cell Sorting System (Miltenyi Biotec, Bergisch Gladbach, Germany).

Separation of mouse fetal fibroblasts: A 13- or 14-day-pregnant female mouse (C57BL/6) was sacrificed by cervical dislocation and cleaned in 70% ethanol. The extracted uterus was transferred to a Petri dish containing 5 ml of a phosphate buffer solution (PBS, Wako Pure Chemical Industries, Osaka, Japan) containing penicillin (final concentration: 100 units/ml), streptomycin (final concentration: 100 µg/ml), and amphotericin B (final concentration: 0.25 µg/ml; Invitrogen, Carlsbad, CA). After separating the fetus from the placenta and cutting off the brain and the heart, it was placed in a 50-ml conical tube, 2 ml of 0.25% trypsin/EDTA (Sigma-Aldrich) was added, and it was shaken at 37°C for 30 minutes. To inactivate the trypsin, 10 ml of base medium was added, and after chopping using cutting shears, 18- and 20-gauge needles were passed through 3 times each using a syringe, and a single cell suspension was prepared through a 100-µm cell strainer (BD Biosciences). After performing centrifugal separation on this cell suspension at 4°C and 1,500 rpm for 5 minutes, the supernatant was disposed, 3 ml of an erythrocyte hemolysis buffer solution (Roche Applied Science) was added, and a reaction was performed for 3 minutes at room temperature. After adding 10 ml of the base medium, performing centrifugal separation at 4°C and 1,500 rpm for 5 minutes, and disposing the supernatant, a similar hemolytic reaction was performed again. Then, 10 ml of the base medium was added, centrifugal separation was performed at 4°C and 1,500 rpm for 5 minutes, the supernatant was disposed, 10 ml of the base medium was added, and a single cell suspension was prepared through a 40-µm cell strainer (BD Biosciences). The 1-2 x 10⁶ cells were seeded and cultured in a 10-cm culture dish coated with 0.1 % (w/v) gelatin. Cells of the first through third passages were frozen and preserved in liquid nitrogen.

Preparation of feeder cells: Feeder cells are mouse fetal fibroblasts for which proliferation has been stopped by mitomycin C. To confluent mouse fetal fibroblasts of the second through fourth passages, 10 ug/ml of mitomycin C (Sigma-Aldrich) diluted by the base medium was added, and culturing was performed for 2 hours at 37°C to prepare feeder cells. The feeder cells were frozen and preserved in liquid nitrogen.

Culturing of lung tissue stem cells: The feeder cells were seeded in a 6-well plate (BD Falcon) at a density of 1 × 10⁴ cells/cm², cultured for 24 hours, and adhered to the bottom surfaces of the wells. Lung cells from which hematopoietic cells had been removed were seeded at a density of 1-5 x 10⁵ cells/cm² in a plate coated with the feeder cells, and cultured in the base medium with 5% CO₂ at 37°C. For the first 7 days, 0.25 µg/ml of amphotericin B was added. After spindle-shaped cells became confluent, the cells were separated using 0.25% trypsin/EDTA (Sigma-Aldrich), and passaged in a 10-cm culture plate coated with the feeder cells. Moreover, when passaging by picking up colonies, 0.01% trypsin/EDTA was added, colonies composed of spindle-shaped cells were separated from the surroundings using a 27-gauge needle and suctioned using a pipette, and seeded in a 6-well plate coated with the feeder cells. From the second passage onward, the culture plate was not coated with the feeder cells, and a feeder cell conditioned medium was used as the medium. The feeder cell conditioned medium was one in which the product of filtering 0.45 µm of the supernatant from culturing the feeder cells for 3 days in the base medium was mixed with the base medium at a ratio of 1:1. This was frozen and preserved at -80°C.

Flow cytometry: For cells of the fourth and fifth passages, the cell surfaces or the intracellular antigens were analyzed using an FACSCalibur flow cytometer (BD Biosciences). The antibodies used were as follows: FITC-labeled anti-human CD45, FITC-labeled anti-human CD105 (Biolegend, San Diego, CA), FITC-labeled anti-human CD31, PE-labeled anti-human CD73, APC-labeled anti-human CD90, PE-labeled anti-human c-kit (BD Biosciences, San Jose, CA), PE-labeled anti-human CD133/1 (Miltenyi Biotec, Bergisch Gladbach, Germany), rabbit anti-human pro SP-C polyclonal antibody (Millipore Corporation, Billerica, MA), and FITC-labeled goat anti-rabbit IgG as second antibody (Vector, Burlingame, CA). When staining the intracellular protein, a fixation and permeabilization kit was used (Immunotech sas, Marseille Cesex 9, France). Normal rabbit IgG (1 µg/ul; Dako, Glostrup, Denmark) was used as an isotype control for SP-C stains.

Fluorescent immunostaining: Cells of the fifth and sixth passages were cultured on a CultureSlide glass (BD Falcon). The human lung tissue was embedded in an OCT compound, and a frozen section was prepared. This was thinly sliced to a thickness of 3 µm in a cryostat. In any case, after fixing for 10 minutes using 100% acetone, blocking was performed for 30 minutes at room temperature using 5% goat serum. Rabbit anti-human pro SP-C polyclonal antibody (1:1000, Millipore Corporation) and mouse anti-human CD90 antibody (1:50, Serotec) were reacted overnight at 4°C, and as a secondary antibody, FITC-goat anti-rabbit IgG (1:100, Vector) and Alexa Fluora 647-goat anti-mouse IgG (1:100, Molecular Probe) were reacted for 30 minutes at room temperature.

Limiting dilution method: For every 1 well, 1, 10 or 100 cells (5th passage) were seeded in a 96-well plate (Coming Incorporated, Coming, NY) and cultured for 14 days in a base medium. The number of cells seeded for every 1 well was plotted on the X-axis, and the proportion of wells that did not form colonies was plotted on the Y-axis. A regression line was drawn, and from the X-axis value corresponding to 37% on the Y-axis, the number of cells including 1 colony-forming cell was obtained (11). For the colonies obtained from wells in which 1 cell was seeded for every 1 well, passage culturing was continued in a feeder conditioned medium. For cells of the seventh passage, the phenotypes of the cell surfaces and the intracellular protein were determined, and they were frozen and preserved.

Differentiation into alveolar epithelial cells: To investigate the capacity to differentiate into alveolar epithelial cells, for every 1 well, 1×10⁶ cells (fifth and sixth passages) were suspended in 1 ml of a frozen base medium, mixed well in 1 ml of Matrigel (trademark) (BD Biosciences) and ice, seeded in a 12-well plate, and gelated over 30 minutes at 37°C. Subsequently, 1 ml of base medium warmed to 37°C was gently inserted into the Matrigel, and culturing was continued at 37°C. After seven days, the cells within the Matrigel and under the Matrigel were each divided and recovered, and RNA was extracted. For the cells within the Matrigel, the Matrigel was crushed using a chilled PBS fluid, and they were cleaned and recovered using the PBS. In this operation, to prevent the cells under the Matrigel from breaking free, the cells under the Matrigel were then suspended in an EDTA/Trypsin fluid and recovered.

Statistical analysis: GraphPad Prism Ver 5.0b (GraphPad Software, San Diego, CA) was used. For the limiting dilution method, the regression formula and R² were obtained using non-linear regression. Regarding the relationship between age and the proportion of SP-C+/CD90+ cells, Spearman's rank relation coefficient was obtained.

### [Results]

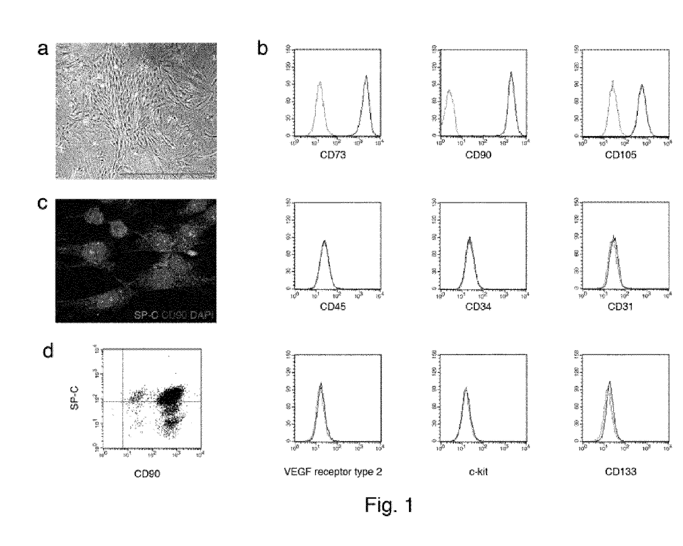

Result 1: In the human lung, cell groups resembling mesenchymal stem cells that have the phenotype of the alveolar epithelium are present.
When human lung constituent cells from which hematopoietic cells had been removed were cultured on a feeder, spindle-shaped cells proliferated and colonies were formed after approximately 7 days (Fig. 1a). These cells could be passaged, and became confluent after 2 to 3 weeks. The expression of these cell surface markers was analyzed using flow cytometry (Fig.1b). Many cells expressed CD73, CD90, and CD 105, which are known as human mesenchymal stem cell markers (12). There was no observed expression of CD45, CD34, CD31, or VEGF receptor type 2, which are markers of blood cells and the vascular endothelium. Moreover, there was also no observed expression of c-kit (13) or CD133 (14), which are tissue stem cell markers. Based on the above findings, the obtained cell groups had surface markers representing mesenchymal stem cells. CD90, which is also known as Thy-1, is a GPI membrane-bound protein, and is known not only as a marker of mesenchymal stem cells, but also as a marker of hematopoietic stem cells expressing CD34 (15) and of liver stem cells (16). Moreover, surfactant protein-C (SP-C) is known to be expressed specifically to type II alveolar epithelial cells (17). Therefore, in order to investigate whether the obtained cell groups have the phenotype of the alveolar epithelium, double staining of SP-C and CD90 was performed using the immunofluorescent antibody method (Fig. 1c). Cells positive for both SP-C and CD90 were observed, and SP-C (green) was stained granularly in the cells while CD90 (red) was stained partially along the cell membrane. Then, in flow cytometry, most of the cells were SP-C+/CD90+ cells (Fig. 1d). Based on the above investigation, it became clear that in the human lung, new cells groups that simultaneously express SP-C, which is a marker of type II alveolar epithelial cells, and CD90, which is a stem cell marker, were present.

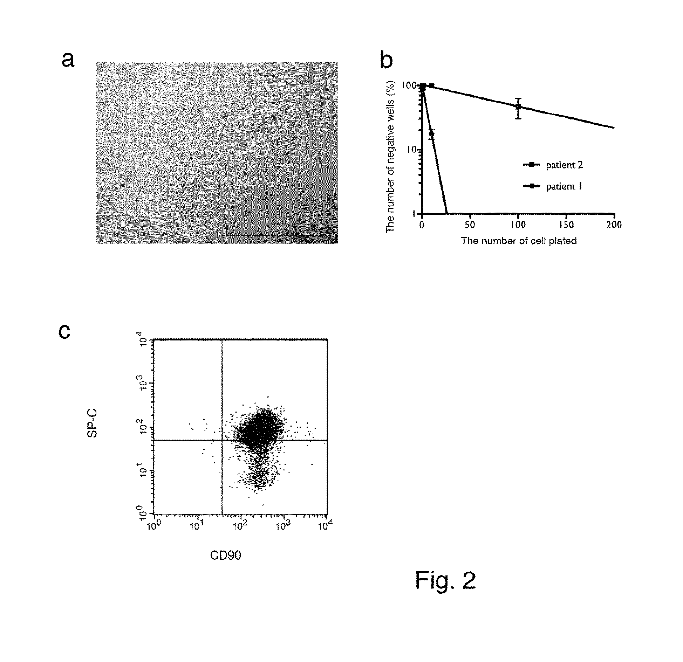

Result 2: SP-C+/CD90+ cells exhibit a self-replicating function in vitro. Tissue stem cells are defined as cells that are separated from the tissue and exhibit a self-replicating function and the capacity to differentiate into more mature cells (18). We believed that SP-C+/CD90+ cells were a candidate for human lung tissue stem cells, and first investigated the self-replicating function. As shown in Fig. 2a, from single cells obtained through limiting dilution, colony formation was observed after 10 days. Next, regarding cell groups containing many SP-C+/CD90+ cells, in order to determine the frequency of cells having a colony-forming ability, the limiting dilution method was performed (Fig. 2b). It was found that cells having a colony-forming ability were contained at a rate of 1 per 6,131 cells in both patients 1 and 2. Regarding secondary colonies formed from wells seeded with 1 cell per well, when the expression of SP-C and CD90 were analyzed using flow cytometry, as shown in Fig. 2c, an expression pattern similar to that of the original cell groups was exhibited. Based on the above findings, it was shown that SP-C+/CD90+ cells have a self-replicating function *in vitro.*

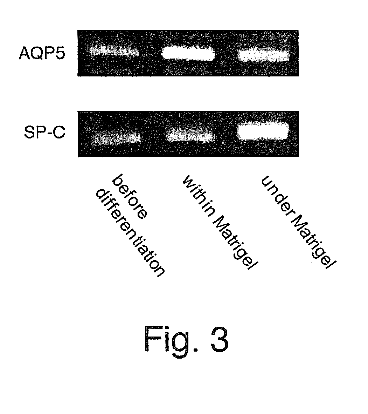

Result 3: SP-C+/CD90+ cells exhibit differentiation into alveolar epithelial cells *in vitro.*
To investigate the capacity of SP-C+/CD90+ cells to differentiate into alveolar epithelial cells, cell groups containing many SP-C+/CD90+ cells were cultured for 7 days in Matrigel, RNA was extracted from cells within the Matrigel as well as cells below the Matrigel, and changes in mRNA expression were investigated using an RT-PCR. As shown in Fig. 3a, within the Matrigel, the expression of AQP5, which is a marker of type I cells, was increased, and under the Matigel, the expression of SP-C, which is a marker of type II cells, was increased. Based on the above findings, the induction of differentiation into type I cells within the Matrigel and into type II cells under the Matrigel was observed.

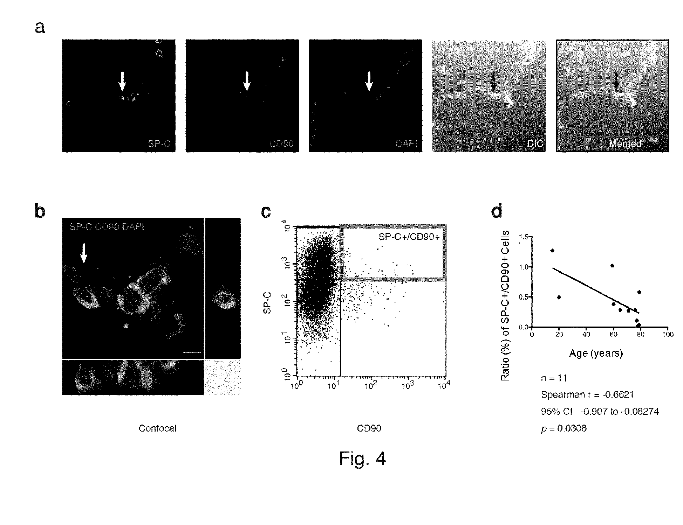

Result 4: SP-C+/CD90+ cells are present in the walls of the alveoli.
To determine the local presence of SP-C+/CD90+ cells, a frozen section of human lung tissue was prepared and fluorescent immunostaining was performed. As shown in Fig. 4a, SP-C+/CD90+ cells were present in the walls of the alveoli. In observations using a confocal laser scanning microscope, SP-C was stained inside the cells and CD90 was observed in part of the cell surface, and a staining pattern identical to that of SP-C+/CD90+ cells separated from the human lung was observed (Fig. 4b). To investigate the proportion of SP-C+/CD90+ cells, lung cells from which hematopoietic cells had been removed were analyzed using flow cytometry. As shown in Fig. 4c, SP-C+/CD90+ cells were contained at a rate of 0.45% ± 0.34% (mean ± S.D.), and it was found to exhibit a significant inverse correlation with age (Fig. 4d). Based on the above investigation, it became clear that SP-C+/CD90+ cells are present in the walls of the alveoli, and that decreases in number are observed with age.

### [References]

1. Adamson, I.Y, and Bowden, D.H. 1974. The type 2 cell as progenitor of alveolar epithelial regeneration. A cytodynamic study in mice after exposure to oxygen. Lab Invest 30:35-42.
2. Evans, M.J., Cabral, L.J., Stephens, R.J., and Freeman, G. 1975. Transformation of alveolar type 2 cells to type 1 cells following exposure to NO2. Exp Mol Pathol 22:142-150.
3. Aso, Y, Yoneda, K., and Kikkawa, Y 1976. Morphologic and biochemical study of pulmonary changes induced by bleomycin in mice. Lab Invest 35:558-568.
4. Anderson, W.R., and Thielen, K. 1992. Correlative study of adult respiratory distress syndrome by light, scanning, and transmission electron microscopy. Ultrastruct Pathol 16:615-628.
5. Ware, L.B., and Matthay, M.A. 2000. The acute respiratory distress syndrome. N Engl J Med 342:1334-1349.
6. Kawanami, O., Ferrans, V.J., and Crystal, R.G. 1982. Structure of alveolar epithelial cells in patients with fibrotic lung disorders. Lab Invest 46:39-53.
7. Kim, C.F., Jackson, E.L., Woolfenden, A.E., Lawrence, S., Babar, I., Vogel, S., Crowley, D., Bronson, R.T., and Jacks, T. 2005. Identification of bronchioalveolar stem cells in normal lung and lung cancer. Cell 121:823-835.
8. Kubo, H., Hegab, A.E., He, M., Ishizawa, K., and Yamada, M. 2008. Endogenous lung stem cells increased after lung injury. Proc Am Thorac Soc 5:362-363.
9. Hegab, A.E., Kubo, H., Yamaya, M., Asada, M., He, M., Fujino, N., Mizuno, S., and Nakamura, T. 2008. Intranasal HGF administration ameliorates the physiologic and morphologic changes in lung emphysema. Mol Ther 16:1417-1426.
10. Bortnick, A.E., Favari, E., Tao, J.Q., Francone, O.L., Reilly, M., Zhang, Y., Rothblat, G.H., and Bates, S.R. 2003. Identification and characterization of rodent ABCA1 in isolated type II pneumocytes. Am J Physiol Lung Cell Mol Physiol 285:L869-878.
11. Tropepe, V., Coles, B.L., Chiasson, B.J., Horsford, D.J., Elia, A.J., McInnes, R.R., and van der Kooy, D. 2000. Retinal stem cells in the adult mammalian eye. Science 287:2032-2036.
12. Dominici, M., Le Blanc, K., Mueller, I., Slaper-Cortenbach, I., Marini, F., Krause, D., Deans, R., Keating, A., Prockop, D., and Horwitz, E. 2006. Minimal criteria for defining multipotent mesenchymal stromal cells. The International Society for Cellular Therapy position statement. Cytotherapy 8:315-317.
13. Bearzi, C., Rota, M., Hosoda, T., Tillmanns, J., Nascimbene, A., De Angelis, A., Yasuzawa-Amano, S., Trofimova, I., Siggins, R.W., Lecapitaine, N., et al. 2007. Human cardiac stem cells. Proc Natl Acad Sci U S A 104:14068-14073.
14. Uchida, N., Buck, D.W., He, D., Reitsma, M.J., Masek, M., Phan, T.V., Tsukamoto, A.S., Gage, F.H., and Weissman, I.L. 2000. Direct isolation of human central nervous system stem cells. Proc Natl Acad Sci U S A 97:14720-14725.
15. Craig, W., Kay, R., Cutler, R.L., and Lansdorp, P.M. 1993. Expression of Thy-1 on human hematopoietic progenitor cells. J Exp Med 177:1331-1342.
16. Herrera, M.B., Bruno, S., Buttiglieri, S., Tetta, C., Gatti, S., Deregibus, M.C., Bussolati, B., and Camussi, G. 2006. Isolation and characterization of a stem cell population from adult human liver. Stem Cells 24:2840-2850.
17. Phelps, D.S., and Floros, J. 1991. Localization of pulmonary surfactant proteins using immunohistochemistry and tissue in situ hybridization. Exp Lung Res 17:985-995.
18. Weiss, D.J., Kolls, J.K., Ortiz, L.A., Panoslcaltsis-Mortari, A., and Prockop, D.J. 2008. Stem cells and cell therapies in lung biology and lung diseases. Proc Am Thorac Soc 5:637-667.

### Industrial Applicability

As a result of the present invention, it has become clear that SP-C+/CD90+ cells are present in human lung tissue, and are human tissue stem cells exhibiting a self-replicating function and the capacity to differentiate into alveolar epithelial cells. Moreover, it was found that the number of SP-C+/CD90+ cells decreases with age. Because these types of stem cells that are able to differentiate into the alveolar epithelial system are cells involved in tissue repair after a lung injury, they are clinically very important cells for regenerative medicine, etc. Furthermore, these types of cells are also useful as materials for discovering new markers for identifying human lung tissue stem cells, and by analyzing the differentiation signals, etc. of the human tissue stem cells obtained in the present invention, it becomes possible to provide new drug discoveries.

## Claims

1. A method of preparing cells that simultaneously express a type II alveolar epithelial cell marker and a stem cell marker, comprising a process of isolating and extracting constituent cells from human lung tissue and a process of separating and culturing lung tissue stem cells from the obtained isolated cells.

2. The method of preparation according to Claim 1, wherein the cells are SP-C⁺/CD90⁺ cells.

3. The method of preparation according to Claim 1 or 2, wherein the cells are human lung tissue stem cells that are able to differentiate into human alveolar epithelial cells.

4. The method of preparation according to any one of Claims 1 through 3, wherein in the process of isolation and extraction, after separating and removing the pleura from the human lung tissue, a neutral protease solution is injected into the lung tissue, followed by dissolving the cells in a solution including collagenase, neutral protease, and deoxyribonuclease.

5. The method of preparation according to any one of Claims 1 through 4 comprising the process of separating and culturing human lung tissue stem cells using feeder cells composed of mouse fetal fibroblasts.

6. Human lung tissue stem cells that are obtained by the method of preparation according to any one of Claims 1 through 5 and are able to differentiate into human alveolar epithelial cells, or human lung tissue stem cells that are obtained by passage culturing the cells.

7. A method of inducing differentiation into human lung epithelial cells, comprising culturing the human lung tissue stem cells according to Claim 6.

8. The method of inducing differentiation according to Claim 7, wherein through culturing using a reconstituted basement membrane composition, type I alveolar epithelial cells are obtained within the reconstituted basement membrane composition, and type II alveolar epithelial cells are obtained from under the reconstituted basement membrane composition.

9. The method of inducing differentiation according to Claim 7 or 8 wherein the reconstituted basement membrane composition is a soluble basement membrane preparation extracted from a mouse sarcoma.

10. Human alveolar epithelial cells prepared by the method of inducing differentiation according to any one of Claims 7 through 9, or human alveolar epithelial cells obtained by passage culturing the human alveolar epithelial cells.

11. A method of screening wherein the human lung tissue stem cells according to Claim 5 or the human alveolar epithelial cells according to Claim 9 are used.
